# EUROPÄISCHE PATENTSCHRIFT

(11) **EP 2 014 278 B1**
(45) Veröffentlichungstag und Bekanntmachung des Hinweises auf die Patenterteilung: **04.05.2016**
(21) Anmeldenummer: 08010803.8
(22) Anmeldetag: 13.06.2008
(51) Int. Cl.: A61Q 19/00, A61Q 19/08, A61K 8/55, A61K 8/60, A61K 8/73, A61K 8/97, A61K 8/04

(54) **Wirkstoffkombination für kosmetische Zubereitungen**
Substance combination for cosmetic preparations
Combinaison de substances pour préparations cosmétiques

(30) Priorität: 15.06.2007 DE 102007028360
(43) Veröffentlichungstag der Anmeldung: 14.01.2009
(73) Patentinhaber: Asam, Marcus, 85774 Unterföhring (DE); Asam, Mirjam, 85774 Unterföhring (DE)
(72) Erfinder: Asam, Marcus, 85774 Unterföhring (DE)
(74) Vertreter: Witzany, Manfred

(56) Entgegenhaltungen:
- WO-A2-2006/020994
- US-A- 5 391 373
- US-A- 5 571 503
- US-A1- 2007 042 030
- "Gfn/Selco. new4you" INTERNET CITATION, [Online] 28. März 2006 (2006-03-28), Seiten 1-5, XP007914003 Gefunden im Internet: URL:http://www.gfn-selco.de/dokumente/InCo smetics_2006_New4You_d01.pdf> [gefunden am 2010-07-19]

## Beschreibung

Die Erfindung betrifft eine Wirkstoffkombination für kosmetische Zubereitungen zur äußerlichen Auftragung auf die Haut, insbesondere Hautcremes.

Aus der WO 2006/020994 A2 ist bekannt, Hyaluronsäure zur Injektion in die Haut als Wirkstoff einzusetzen. Hyaluronsäure kann ein Vielfaches des eigenen Molekulargewichts an Wasser binden und bildet daher in der Haut einen wichtigen Feuchtigkeitsspeicher. Hyaluronsäure wird in den Hautzellen ständig gebildet, wobei die natürliche Hyaluronsäuresynthese mit zunehmendem Alter abnimmt, was eine der wesentlichen Ursachen der Faltenbildung in der Haut ist. Durch die Injektion von Hyaluronsäure wird der Faltenbildung entgegengewirkt.

Aus der US-A-2007/0042030 ist bekannt, Wirkstoffe in Phosphorlipid-Membranen einzuhüllen, damit sie leichter durch Barrieren dringen können. Derartige Barrieren ergeben sich beispielsweise in den obersten Hautschichten.

Aus der XP-007914003 ist eine gattungsgemäße Wirkstoffkombination bekannt. Sie enthält Hyaluronsäure in verschiedenen Molekulargewichtsbereichen, die unterschiedlich wirksam sind. Eine hochmolekulare Hyaluronsäure mit einem Molekulargewicht von mehr als 1,8 MDalton wird als Formbildner und Feuchtigkeitsspender eingesetzt. Zusätzlich wird vorgeschlagen, eine kurzkettige Hyaluronsäure in einem Molekulargewichtsbereich von 0,01 - 0,15 MDalton einzusetzen, die besser in die Haut penetriert. Dies wirkt insbesondere dem Alterungsprozess der Haut entgegen. Diese Wirkstoffformulierung bildet den Ausgangspunkt der vorliegenden Erfindung.

Der Erfindung liegt die Aufgabe zugrunde, eine Wirkstoffkombination der eingangs genannten Art zu schaffen, die sich durch eine verbesserte Tiefenwirkung in der Haut auszeichnet.

Diese Aufgabe wird erfindungsgemäß mit den Merkmalen des Patentanspruchs 1 gelöst.

Die Wirkstoffkombination gemäß Anspruch 1 weist Hyaluronsäure und/oder mindestens ein Hyaluronsäurederivat auf. Dieses soll die natürlichen Hyaluronsäuredepots in der Haut ergänzen, um Alterserscheinungen wie Faltenbildung vorzubeugen. Hyaluronsäure ist ein Polymer, welches in unterschiedlichen Molekulargewichten eingesetzt werden kann. Je höher das Molekulargewicht der Hyaluronsäure ist, umso besser ist die Wasserspeicherwirkung. Diese erhöhte Wasserspeicherwirkung geht jedoch auf Kosten der Tiefenwirkung der Hyaluronsäure, da größere Moleküle zwangsläufig nur eine geringe Eindringtiefe in die Haut aufweisen. Langkettige Hyaluronsäure bleibt daher im wesentlichen auf der Hautoberfläche liegen, so daß sie keine Tiefenwirkung entfalten kann. Kurzkettige Hyaluronsäuremoleküle besitzen zwar eine geringere Wasserspeicherfähigkeit, sie können jedoch im Gegensatz zu den langkettigen Formen tiefer in die Haut eindringen. Erfindungsgemäß werden langkettige Hyaluronsäure bzw. deren Derivate mit einem Molekulargewicht von mindestens einer Million Dalton mit kurzkettiger Hyaluronsäure bzw. deren Derivaten mit einem Molekulargewicht von höchstens zweihunderttausend Dalton in Kombination eingesetzt. Die Masseneinheit Dalton entspricht einem zwölftel der Masse des Kohlenstoffisotops ¹²C und entspricht in etwa der Atomaren Masseneinheit. Durch diese Kombination konnte überraschenderweise eine höhere Wasserabgabe in tiefen Hautschichten nachgewiesen werden, als dies allein mit kurzkettigen Hyaluronsäuren möglich ist. Es wird angenommen, daß die langkettigen Hyaluronsäuremoleküle an der Hautoberfläche einen positiven Einfluß auf die physikalischen Eigenschaften der kurzkettigen Hyaluronsäuremoleküle in tieferen Hautschichten haben. Konkret geklärt ist dieser Mechanismus jedoch noch nicht. Die angegebenen Mindestwerte für die Anteile an langkettigen und kurzkettigen Formen der Hyaluronsäure bzw. -derivate beziehen sich stets auf die Wirkstoffkombination und nicht auf die gesamte kosmetische Zubereitung. Dies bedeutet, daß in einer realen Wirkstoffkombination die Summe aller Hyaluronsäuren bzw. -derivate stets 100 Gewichtsprozent ausmacht. Weitere Stoffe, die regelmäßig in kosmetischen Zubereitungen enthalten sind, wie beispielsweise Glyzerin usw. sind bei der Bestimmung der Gewichtsprozente der Wirkstoffkombination selbst nicht zu berücksichtigen. Schließlich enthält die Wirkstoffkombination Aescin einen Komplex aus dem Phytowirkstoff der Roßkastanie. Dieser Wirkstoff unterdrückt die Depolimerisation der Hyaluronsäure, so daß das chemische Gleichgewicht zu längerkettigen Hyaluronsäuren verschoben ist. Außerdem bleiben Hyaluronsäuremoleküle in den tieferen Hautschichten länger erhalten und wirksam. Um die Hyaluronsäure bzw. das Hyaluronsäurederivat möglichst effektiv in tiefere Hautschichten einbauen zu können, ist diese wenigstens teilweise von Phosphorlipiden eingehüllt. Die Oberfläche des Hyaluronsäuremoleküls bzw. dessen Derivats wird dabei von den Phosphorlipiden verdeckt, so daß die nach außen hin wirksame Oberfläche der eines Lipids entspricht. Damit kann die Hyaluronsäure bzw. deren Derivat unter Zuhilfenahme hydrophober Kräfte besonders effektiv in die Haut eindiffundieren.

Neben reiner Hyaluronsäure hat sich auch Alkalimetall substituierte Hyaluronsäure bewährt. Vorzugsweise wird dabei ein Wasserstoff der Karbonatgruppe (COOH) durch ein Alkalimetall substituiert. Als Substituent hat sich insbesondere das reaktionsfreudige Natrium bewährt. Alternativ kann die Hyaluronsäure auch mit wenigstens einer Alkylthiosulfatgruppe substituiert sein. Diese funktionelle Gruppe bildet sehr leicht Disulfidbrücken, die zur stereoisomeren Stabilisierung des Hyaluronsäurederivats beitragen. Schließlich kann die Hyaluronsäure auch mit wenigstens einer Silangruppe substituiert sein.

Durch einen mindestens 20 %igen Gehalt an langkettiger Hyaluronsäure bzw. -derivat gemäß Anspruch 2 ist eine hohe Wasserspeicherfähigkeit sichergestellt, so daß die Wirkstoffkombination insgesamt sehr effektiv wirkt.

Um insbesondere tiefgelegene Hautschichten zu erreichen, ist es gemäß Anspruch 3 günstig, wenn von der kurzkettigen Form der Hyaluronsäure bzw. -derivat mindestens b Gewichtsprozent enthalten sind. Dies ist insbesondere zur Behandlung tiefer Falten bedeutend.

Um zu verhindern, daß die Hyaluronsäure bzw. deren Derivats aufgrund der umhüllenden Phosphorlipide und des damit vergrößerten Durchmessers von der Haut abgeblockt wird, ist es gemäß Anspruch 4 günstig, wenn die Hyaluronsäure bzw. dessen Derivat ein Molekulargewicht von höchstens 150.000 Dalton hat.

### Beispiele

Eine Wirkstoffkombination besteht aus:
43 Gewichtsprozent langkettiger natriumsubstituierter Hyaluronsäure mit einem Molekulargewicht von ca. 1,5 Millionen Dalton;
10 Gewichtsprozent kurzkettiger natriumsubstituierter Hyaluronsäure mit einem Molekulargewicht von ca. 100.000 Dalton;
42 Gewichtsprozent eines kurzkettigen Silanolderivats der Hyaluronsäure mit einem Molekulargewicht von ca. 100.000 Dalton, welche in Phosphorlipiden eingehüllt ist und 5 Gewichtsprozent Aescin, einem Komplex aus dem Phytowirkstoff der Roßkastanie.

Die oben genannte Wirkstoffkombination wird mit Wasser angesetzt, wobei 5,5 % Wirkstoffkombination, 5 % Glyzerin und 89,5 % Wasser enthalten sind. Dabei ergibt sich ein Gel mit einer Viskosität, die ein Pumpen in üblichen Gelspendern ermöglicht. Wird dieses Gel in Falten der Haut eingetragen, so ergibt sich sofort ein glättender Effekt. Außerdem ergibt sich bereits nach wenigen Minuten eine nachweisbare Befeuchtung der tieferen Hautschichten, die über die Summe der Wirksamkeit der einzelnen Wirkstoffe hinausgeht.

In einer weiteren Formulierung werden 5,5 % der Wirkstoffkombination mit 5 % Glyzerin, 60 % Wasser, 30 % Ölen bzw. Wachsen und 4,5 % Emulgatoren sowie sonstige Zusatzstoffe vermischt. Dabei ergibt sich eine leicht auf der Haut verteilbare und in diese einziehende Creme, mit der der Haut großflächig Feuchtigkeit zugeführt werden kann.

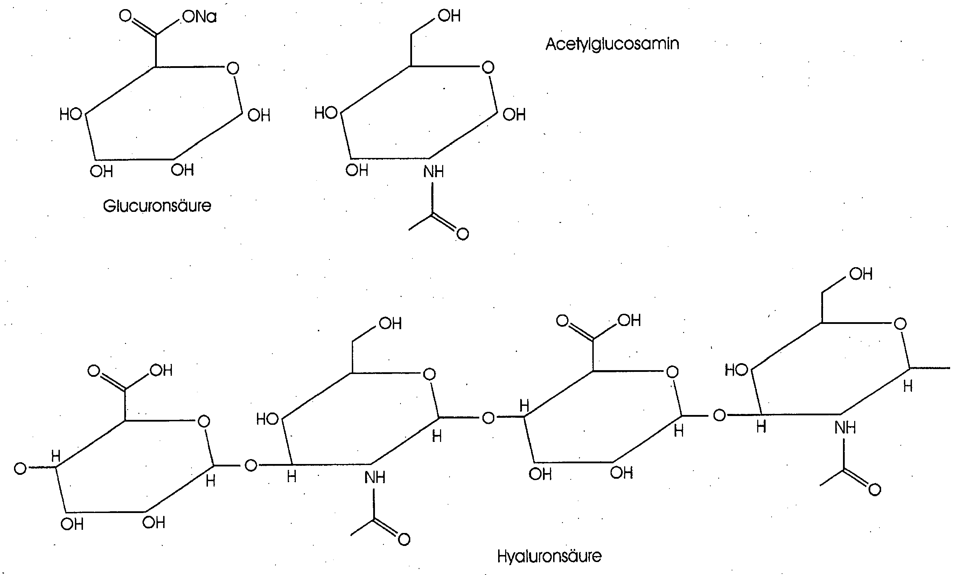

## Patentansprüche

1. Wirkstoffkombination für kosmetische Zubereitungen zur äußerlichen Auftragung auf die Haut, wobei die Wirkstoffkombination Aescin aus Aesculus Hippocastanum aufweist und die Wirkstoffkombination Hyaluronsäure und/oder mindestens ein Hyaluronsäurederivat mit folgenden Parametern aufweist und die Hyaluronsäure und/oder das mindestens eine Hyaluronsäurederivat wenigstens teilweise in Phosphorlipiden eingehüllt ist:
a: mindestens 10 Gewichtsprozent in langkettiger Form mit einem Molekulargewicht von mindestens 1.000.000 Dalton und
b: mindestens 2 Gewichtsprozent in kurzkettiger Form mit einem Molekulargewicht von höchstens 200.000 Dalton,
wobei das mindestens eine Hyaluronsäurederivat eine Alkalimetall, Alkylthiosulfat oder Silan substituierte Hyaluronsäure ist.

2. Wirkstoffkombination nach Anspruch 1, bei der die langkettige Form zu mindestens 20 Gewichtprozent enthalten ist.

3. Wirkstoffkombination nach Anspruch 1 oder 2, bei der die kurzkettige Form zu mindestens 5 Gewichtsprozent enthalten ist.

4. Wirkstoffkombination nach mindestens einem der Ansprüche 1 bis 2, bei der die eingehüllte Form ein Molekulargewicht von höchstens 150.000 Dalton aufweist.

5. Wirkstoffkombination nach mindestens einem der Ansprüche 1 bis 4, bei der das Hyaluronsäurederivat mindestens ein Natriumatom enthält.

## Claims

1. Active ingredient combination for cosmetic preparations for external application to the skin, where the active ingredient combination has aescin from Aesculus Hippocastanum and the active ingredient combination has hyaluronic acid and/or at least one hyaluronic acid derivative with the following parameters and the hyaluronic acid and/or the at least one hyaluronic acid derivative is coated at least partially in phospholipids:
a: at least 10 percent by weight in long-chain form with a molecular weight of at least 1 000 000 daltons and
b: at least 2 percent by weight in short-chain form with a molecular weight of at most 200 000 daltons,
where the at least one hyaluronic acid derivative is an alkali metal-, alkyl thiosulfate- or silane-substituted hyaluronic acid.

2. Active ingredient combination according to Claim 1, in which the long-chain form is present to at least 20 percent by weight.

3. Active ingredient combination according to Claim 1 or 2, in which the short-chain form is present to at least 5 percent by weight.

4. Active ingredient combination according to at least one of Claims 1 to 2, in which the coated form has a molecular weight of at most 150 000 daltons.

5. Active ingredient combination according to at least one of Claims 1 to 4, in which the hyaluronic acid derivative comprises at least one sodium atom.

## Revendications

1. Combinaison d'agents actifs pour préparations cosmétiques pour l'application extérieure sur la peau, la combinaison d'agents actifs comprenant de l'aescine issue d'Aesculus Hippocastanum et la combinaison d'agents actifs comprenant de l'acide hyaluronique et/ou au moins un dérivé d'acide hyaluronique ayant les paramètres suivants, et l'acide hyaluronique et/ou ledit au moins un dérivé d'acide hyaluronique étant au moins partiellement encapsulé dans des phospholipides :
a : au moins 10 pour cent en poids sous forme à chaîne longue ayant un poids moléculaire d'au moins 1 000 000 Dalton, et
b : au moins 2 pour cent en poids sous forme à chaîne courte ayant un poids moléculaire d'au plus 200 000 Dalton,
ledit au moins un dérivé d'acide hyaluronique étant un acide hyaluronique substitué par un métal alcalin, un thiosulfate d'alkyle ou un silane.

2. Combinaison d'agents actifs selon la revendication 1, dans laquelle la forme à chaîne longue est contenue à hauteur d'au moins 20 pour cent en poids.

3. Combinaison d'agents actifs selon la revendication 1 ou 2, dans laquelle la forme à chaîne courte est contenue à hauteur d'au moins 5 pour cent en poids.

4. Combinaison d'agents actifs selon au moins l'une quelconque des revendications 1 à 2, dans laquelle la forme encapsulée présente un poids moléculaire d'au plus 150 000 Dalton.

5. Combinaison d'agents actifs selon au moins l'une quelconque des revendications 1 à 4, dans laquelle le dérivé d'acide hyaluronique contient au moins un atome de sodium.
